# FASCICULE DE BREVET EUROPEEN

(11) **EP 1 615 632 B1**
(45) Date de publication et mention de la délivrance du brevet: **06.12.2006**
(21) Numéro de dépôt: 04728127.4
(22) Date de dépôt: 18.04.2004
(51) Int. Cl.: A61K 31/205, A61K 9/52, A61K 9/56, A61K 9/58

(54) **FORMULATIONS ORALES FLOTTANTES POUR LA LIBERATION CONTROLEE DE LA BETAINE**
SCHWIMMENDE ORALE FORMULIERUNGEN MIT KONTROLLIERTER FREISETZUNG VON BETAIN
FLOATING ORAL FORMULATIONS FOR CONTROLLED RELEASE OF BETAINE

(30) Priorité: 17.04.2003 BE 200300248
(43) Date de publication de la demande: 18.01.2006
(73) Titulaire: Messadek, Jallal, 4000 Liège (BE)
(72) Inventeur: Messadek, Jallal, 4000 Liège (BE)
(86) Numéro de dépôt international: PCT/BE2004/000053
(87) Numéro de publication internationale: WO 2004/091601

(56) Documents cités:
- WO-A-02/00213
- WO-A-02/062322

## Description

Le but de la présente invention est une forme à libération contrôlée flottante destinée a libérer au moins une bétaine après administration orale. L'administration orale d'une bétaine contenue dans une forme pharmaceutique solide, semi solide ou liquide à libération contrôlée permettant d'obtenir les taux sanguins désirés et de les maintenir stables pendant une période prolongée. Le but de la présente invention est une forme matricielle flottante destinée à augmenter le temps moyen de résidence gastrique au moins d'une bétaine afin d'en améliorer la biodisponibilité, d'augmenter le passage de cette bétaine vers le sang et permettre le contrôle prolongé des profils plasmatiques. Le but de la présente invention est également un procédé et/ou un système permettant la réalisation d'au moins un des points suivants:
a) d'augmenter le temps de résidence dans le tractus gastro-intestinal d'au moins une bétaine
b) de contrôler et/ou augmenter en fonction du temps le relargage d'au moins une bétaine vers le flux sanguin
c) de contrôler et/ou augmenter la quantité relarguée d'au moins une bétaine vers le flux sanguin
d) de contrôler et/ou augmenter la biodisponibilité d'au moins une bétaine

### Etat de la technique

Les bétaines (CH₃)₃N⁺- (CH₂)ₙ - COO⁻ n étant un entier de 1 à 5, sont des molécules connues pour leurs propriétés osmoprotectrices ainsi que pour ses utilisations cosmétiques et pharmaceutiques. Diverses applications pharmaceutiques de la bétaine sont connues et en particulier l'utilisation de la bétaine pour le traitement de l'homocystinurie. L'homocystinurie est due à des taux élevés d'homocystéine dans le plasma des patients atteints. L'administration de la bétaine permet d'abaisser la concentration d'homocystéine dans le sang. Les patients homocystinuriques pour près de la moitié sont traités avec de hautes doses orales de bétaine de 6 gr. à 20 gr. par jour. Ces doses élevées de bétaine sont nécessaires afin d'atteindre des concentrations plasmatiques chez les patients de l'ordre de 200 à 400 µMol/L (An indirect response model of homocysteine suppression by betaine: optimising the dosage regimen of betaine in homocystinuria, Angela Matthews et al, Br J Clin Pharmacol, 54, 140-146).

Après administration orale la biodisponibilité absolue de la bétaine apparaît comme étant très faible de l'ordre de 10 à 15 % (Schwahn BC et al : Pharmacokinetics of oral betaine in healthy subjects and patients with homocystinuria. Br J Clin Pharmacol 2003 Jan;55(1):6-13).

La bétaine pour traiter l'homocystinurie est disponible sous la forme d'un médicament le Cystadane®. La posologie habituelle chez les patients adultes et les jeunes patients est de 6 grammes par jour, administrés par voie orale, en doses fractionnées de 3 grammes, deux fois par jour. La quantité prescrite de Cystadane® (poudre anhydre de bétaine pour solution orale) devrait être calculée au moyen de la cuillère à mesurer fournie (la mesure à niveau unique de 1,7 cc est égale à 1 gramme de poudre anhydre de bétaine) et ensuite dissoute dans 4 à 6 onces d'eau, de jus, de lait ou de lait maternisé ou mélangée à des aliments pour ingestion immédiate (Monographie du Cystadane®). On ne connaît pas d'autres méthodes d'administration par voie orale de la bétaine et il apparaît que les grandes quantités prescrites (jusqu'à 20 grammes par jour) reflètent la faible biodisponibilité de la bétaine.

Lorsque l'on se penche sur les profils plasmatiques de la bétaine après administration par voie orale chez l'humain, il apparaît que celle-ci se retrouve très vite dans le sang pour être rapidement distribuée dans différents organes. Cette rapide baisse du taux de bétaine dans le sang n'est pas désirable eu égard au fait que ses principaux sites d'action se situent dans la circulation. D'autre part la bétaine est naturellement présente dans les liquides physiologiques et n'est donc pas un xénobiotique. Etant de bas poids moléculaire et naturellement présente dans le corps on pouvait s'attendre à une meilleure biodisponibilité. D'autre part des études menées quant au site d'absorption de la bétaine dans le tractus gastro-intestinal laissent apparaître que la bétaine est absorbée par le duodénum et le jéjunum (H. Kettunen et al, Intestinal uptake of betaine in vitro and the distribution of methyl groups from betaine, choline, and methionine in the body of broiler chicks. Comparative Biochemistry and Physiology Part A 128 2001 269 278).
Il semble donc que la cause de la faible biodisponibilité orale de la bétaine au niveau intestinal soit bien due à une résorption essentiellement limitée au niveau des parties les plus proximales de l'intestin grêle. Une autre cause limitative pourrait être la saturation intestinale, ce qui entraîne le fait que la bétaine au-delà d'un certain seuil ne puisse plus être absorbée. Dès lors l'utilisation de formes flottantes semble bien la meilleure voie possible pour optimiser la résorption intestinale de cette substance. L'autre voie consisterait à augmenter l'adhérence de la bétaine au tractus gastro-intestinal afin de prolonger sa résidence à l'intérieur dudit tractus.

### But de l'invention

Au vu des propriétés pharmacologiques remarquables des bétaines, notamment découvertes par l'inventeur et décrites dans les demandes de brevets WO 00/ 51596, WO 02/ 062322 et PCT/ IB 02/ 04923, ainsi que les publications "Betaine supplementation decreases plasma homocysteine concentrations but does not affect body weight, body composition, or resting energy expenditure in human subjects Ursula Schwab et al , Am J Clin Nutr 2002;76:961" où la bétaine réduit la pression sanguine, notamment la pression diastolique, ainsi que dans la publication "Betaine, a Promising New Agent for Patients With Nonalcoholic Steatohepatitis: Results of a Pilot Study Manal F. Abdelmalek et al, The American Journal Of Gastroenterology Vol. 96, No. 9, 2001" où la bétaine permet de traiter certaines formes d'hépatite, il était intéressant de disposer d'une formulation thérapeutique par voie orale destinée à augmenter la biodisponibilité absolue de la bétaine afin de réduire la quantité ingérée tout en maintenant des taux plasmatiques constants de bétaine pendant des périodes prolongées.

La présente invention décrit ces formes et méthodes notamment, l'utilisation de formes flottantes ayant une densité inférieure à celle des sucs gastriques. On attend des formes flottantes qu'elles se maintiennent durablement au-dessus du contenu stomacal et augmentent par conséquent la biodisponibilité des principes actifs absorbés dans la portion proximale du tractus. Ces formes représentent également le véhicule approprié des principes actifs devant agir localement dans l'estomac nommément au moins une bétaine.

Les formes pharmaceutiques revendiquées peuvent également appartenir à la classe des matrices hydrophiles monolithiques. Ces formes matricielles sont non-désintégrantes et non-digérables. La formulation homogène, contenue au départ dans une capsule de gélatine dure, est préparée à partir d'un ou plusieurs polymères hydrophiles. Ce type de polymère s'hydrate progressivement au contact de l'eau, gonfle et construit une barrière gélifiée superficielle. La présente invention revendique l'utilisation de tout polymère connu et utilisé par l'état de la technique pour réaliser une forme pharmaceutique avantageusement solide à libération contrôlée et/ou une forme flottante pouvant être matricielle ou non destinée(s) à libérer au moins une bétaine après administration orale, de préférence une forme flottante à libération contrôlée. Au moins une bétaine en tant que principe actif est libérée de la matrice, de manière contrôlée et prolongée, principalement par diffusion et érosion en surface.

Le terme bétaine et/ou bétaines dans le champ de la présente invention fait référence aux bétaines décrites et revendiquées dans les applications WO 00/ 51596, WO 02/062322 et PCT/ IB 02/ 04923 de l'inventeur. Les bétaines ainsi que les combinaisons de bétaines entre elles, et les combinaisons de bétaines avec d'autres molécules décrites dans ces applications, pourront dans le champ de la présente invention être formulées, manufacturées, synthétisées, combinées et présentées selon l'invention en une forme flottante à libération contrôlée ou non.

La forme flottante à libération contrôlée peut également être bicouche, une couche contenant au moins une bétaine et l'autre couche destinée à augmenter la flottabilité de ladite forme. La forme flottante à libération contrôlée peut également être multicouches, c'est-à-dire comportant plusieurs couches d'au moins une bétaine séparées par plusieurs couches destinées à augmenter la flottabilité de ladite forme, ces dernières pouvant ou non comporter des indices de dissolution différents et/ou réagir à des pH différents et/ou avoir des indices de relargage d'au moins une bétaine vers le milieu différents.

La forme flottante à libération contrôlée peut également être multicouches c'est-à-dire comportant plusieurs couches d'au moins une bétaine séparées par plusieurs couches destinées à augmenter la flottabilité de ladite forme, ces dernières pouvant ou non comporter des indices de dissolution différents et dans un aspect particulier de l'invention pouvant contenir au moins un autre agent thérapeutique que la bétaine, et optionnellement plusieurs autres agent thérapeutiques. Ces agents thérapeutiques pouvant être à libérations contrôlées ou immédiates et/ou sous des formes flottante s ou non.

La forme flottante, comprenant au moins une couche de libération d'au moins une bétaine et au moins une autre couche destinée à augmenter la flottabilité de ladite forme, étant destinée à augmenter le temps de résidence gastrique d'au moins une bétaine afin que substantiellement cette bétaine soit libérée dans le tractus gastro-intestinal pendant une période étendue.

Dans un aspect de l'invention, la forme flottante comprendra tout polymère physiologiquement acceptable, de préférence un polymère hydrophile, qui au contact des liquides et/ou des fluides gastriques formera une barrière gélatineuse et/ou une barrière gélifiée dont la densité sera inférieure à celle des fluides gastriques et/ou du contenu gastrique. La composition destinée à délivrer au moins une bétaine pouvant être sous forme de capsules, de microcapsules, de comprimés, de tablettes, de liquide, de gel ou de paillettes.

Dans un aspect de l'invention, la forme flottante comprendra tout polymère physiologiquement acceptable qui pourra être séparé d'une bétaine par un film barrière non ionique.
Dans un aspect de l'invention, la forme flottante comprendra plusieurs polymères et/ou des formes galéniques où le et/ou les produits actifs sont dispersés dans un mélange de deux ou plusieurs types de polymères.
Dans un aspect de l'invention, la forme flottante comprendra tout excipient connu de l'homme de l'art qui permet une meilleure absorption gastro-intestinale tels que les sels de bicarbonate, de sodium, de phosphate, etc., ainsi que leurs précurseurs, esters et sels pharmaceutiquement acceptables.

Dans un autre aspect de l'invention les bétaines pourront être synthétisées avec ces excipients, connus selon l'état de la technique, pour augmenter l'absorption gastro-intestinale ainsi que la biodisponibilité.

Dans un autre aspect de l'invention les bétaines pourront être synthétisées ou combinées avec toutes les substances et excipients, connus selon l'état de la technique, pour augmenter l'adhérence aux parois gastro-intestinales.

### Manières de réaliser l'invention

Dans la présente invention les formes flottantes à libération(s) contrôlée(s) destinées à libérer au moins une bétaine après administration orale pourront utiliser les formes et techniques décrites dans les documents suivants:
- les formes décrites dans les US Patent. N°. 4,871,548; 4,767,627; 5,443,843; 5,007,790; 5,582,873; 4,851,232; WO 99/07342. Dans ces documents, les celluloses hydroxyalkylées sont utilisées comme agents gonflants afin d'augmenter le temps de résidence. Ces formes pourront être utilisées selon la présente invention afin de libérer de manière contrôlée au moins une bétaine.
- une autre possibilité pour étendre le temps de résidence dans l'estomac sera de produire des formes flottantes. Celles-ci flottent sur les contenus de l'estomac et, parce que le pylore est localisé dans la partie plus basse de l'estomac, ne sont pas déchargées dans le petit intestin pour une très longue période. Divers procédés sont connus pour produire telles formes. Ainsi, il est possible d'incorporer des substances qui ont une basse densité, telles que, par exemple des graisses, des huiles ou des cires. De telles formes sont décrites dans EP 198769, US Patent. No 4,424,235, US Patent. No 3,343,47, US Patent. No 3,014,98, et BE 839604. Ces formes pourront être utilisées selon la présente invention afin de libérer de manière contrôlée au moins une bétaine.
- Les mélanges dans DE 3527852 qui décrit des mélanges gras contenant qui sont emballés dans une capsule et sont chauffés pour solidification. Ces formes pourront être utilisées selon la présente invention afin de libérer de manière contrôlée au moins une bétaine.
- Les formes enrobées telles que produites dans US Patent. No 4,814,179 où les formes sont refroidies, mises en forme et séchées. Ces formes pourront être utilisées selon la présente invention afin de libérer de manière contrôlée au moins une bétaine.
- Une autre méthode consiste en l'utilisation des gaz issus des sels d'acide carbonique. Ceci signifie que ces sels sont incorporés avec le gel dans les formes de dosage et, après l'exposition à l'acide gastrique du CO₂ est produit qui gonfle la forme, réduit sa densité et entraîne sa flottaison. Dans ce cas on peut incorporer des acides physiologiquement tolérés tels que, par exemple, l'acide citrique, acide ou tartrique. Ces préparations sont emballées dans les capsules de gélatine dures ou douces et sont décrites dans les documents GB 2283172 et GB 2283171 et peuvent servir à libérer au moins une bétaine. Ces formes pourront être utilisées selon la présente invention afin de libérer de manière contrôlée au moins une bétaine.
- La production de tablettes et comprimés est décrite dans WO 99/45887 et US Patent. No 4,167,558. La production de tablettes bicouches est décrite dans les US Patent. No 4,140,755. Ces tablettes produisent un gel après dissolution, gel qui flotte dans l'estomac et libère les principes actifs, ici au moins une bétaine. Ces formes pourront être utilisées selon la présente invention afin de libérer de manière contrôlée au moins une bétaine.
- Les préparations dans lesquelles les couches formant les revêtements sont appliquées à un noyau capable de dégager du CO₂. Dans certains cas, le revêtement lui-même contient un sel d'acide carbonique. De telles préparations sont décrites dans EP 235718, US Patent. No 4,101,650, et WO 99/49868 et peuvent servir à libérer de manière contrôlée au moins une bétaine. Ces formes pourront être utilisées selon la présente invention afin de libérer de manière contrôlée au moins une bétaine.
- Les poudres qui comprennent le principe actif, un hydrocolloide, un polymère pH dépendant, un binder le tout étant emballé dans une capsule sont décrites dans US Patent. No 5,169,638. Ces formes pourront être utilisées selon la présente invention afin de libérer de manière contrôlée au moins une bétaine.
- US Patent. No 5,232,704 décrit des formes consistant en 2 couches, celle contenant le principe actif, et une autre couche responsable de la flottaison. Un tel système pouvant servir à délivrer au moins une bétaine et comportant une couche contenant le principe actif, et une autre couche responsable de la flottaison. Ce système pouvant également s'appliquer à des formes multicouches où plusieurs couches responsables de la flottaison pourra être utilisé pour la bétaine en enrobant successivement plusieurs couches d'au moins une bétaine ou comprenant au moins une bétaine.
- Les formes aérogels, les mousses et microcapsules contenant de l'air telles que décrites dans WO 96/25950, EP 326816, et WO 95/05809 peuvent également servir dans le cadre de la présente invention. Ces formes pourront être utilisées selon la présente invention afin de libérer de manière contrôlée au moins une bétaine.
- WO 0990112 décrit des préparations pharmaceutiques effervescentes comprenant un ou des excipients effervescents et plusieurs unités individuelles comprenant un composé pharmaceutiquement actif et optionnellement des excipients où les unités sont fournies avec un système engendrant la flottaison, ledit système comprennent au moins deux couches de revêtement, l'une engendrant un gaz et l'autre couche étant une couche de barrière comprenant le gaz engendré. Un tel système pouvant servir à délivrer au moins une bétaine. Ces formes pourront être utilisées selon la présente invention afin de libérer de manière contrôlée au moins une bétaine.
- WO 02102415 décrit une composition pharmaceutique flottante, qui comprend un ou plusieurs agents thérapeutiques, ici au moins une bétaine, une enveloppe gélifiée obtenue à partir d'une poudre issue des semences de Sativum Lepidium, un ou plusieurs activateurs d'absorption, un ou plusieurs composés générateurs de gaz et des excipients pharmaceutiquement acceptables. Les activateurs d'absorption pouvant être de la gomme de xanthan, de la gomme de karaya ainsi que des composés similaires. La composition destinée à délivrer au moins une bétaine pouvant être sous forme de capsules, de tablettes, de liquide, de gel, de paillettes et/ou des combinaisons de ces formes. Cette composition permettant l'absorption d'au moins une bétaine d'une manière prédominante dans les parties supérieures du tractus gastro-intestinal. Ces formes pourront être utilisées selon la présente invention afin de libérer de manière contrôlée au moins une bétaine.
- EP 1138320 décrit un mélange formulé d'acétate de polyvinyle et de polyvinylpyrrolidone ainsi que des excipients pouvant être utilisés afin de permettre, selon la présente invention, la production d'une forme flottante à libération contrôlée d'au moins une bétaine. Ces formes peuvent être produites par les procédés simples et montrent des forces mécaniques exceptionnelles. Ces formes pourront être utilisées selon la présente invention afin de libérer de manière contrôlée au moins une bétaine.
- Dans le cadre de la présente invention les formes flottantes comportant plusieurs compartiments enrobés par un ou plusieurs polymères et créant un relargage pulsatile tel que décrit dans Accudep® cores (Delsys Corp.) pourra être utilisé pour augmenter le temps de résidence dans le tractus gastro-intestinal, d'au moins une bétaine. Ces formes pourront être utilisées selon la présente invention afin de libérer de manière contrôlée au moins une bétaine.

Aucun de ces documents n'enseigne une forme à libération contrôlée flottante ou apte à devenir flottante, cette forme étant destinée à libérer de manière contrôlée au moins une bétaine en tant que principe thérapeutiquement actif (en particulier pour le traitement de troubles cardiovasculaires, des troubles thrombotiques, etc. et/ou pour prévenir de tels troubles), après administration orale.

Aucun de ces documents n'enseigne une forme à libération contrôlée flottante destinée à augmenter la biodisponibilité absolue d'au moins une bétaine en tant que principe thérapeutiquement actif, après administration orale.
Aucun de ces documents ne revendique une forme à libération contrôlée flottante destinée à augmenter la biodisponibilité absolue d'au moins une bétaine en tant que principe thérapeutiquement actif, après administration orale.
Aucun de ces documents n'enseigne une forme à libération contrôlée flottante destinée à augmenter le temps de résidence dans le tractus gastro-intestinal d'au moins une bétaine.
Aucun de ces documents n'enseigne une forme à libération contrôlée flottante destinée à contrôler et/ou augmenter en fonction du temps le relargage d'au moins une bétaine vers le flux sanguin.
Aucun de ces documents n'enseigne une forme à libération contrôlée flottante destinée à contrôler et/ou augmenter la quantité relarguée d'au moins une bétaine vers le flux sanguin.

Le but de la présente invention est une forme à libération contrôlée flottante ou apte à devenir flottante dans l'organisme, destinée a libérer au moins une bétaine après administration orale.

L'invention a donc entre autre pour objet une formulation pharmaceutique orale à libération contrôlée destinée a libérer au moins une bétaine après administration orale,
caractérisée en ce que ladite formulation comprend ou est associée à au moins un moyen pharmaceutiquement acceptable assurant une flottaison d'au moins une bétaine dans le tractus gastro-intestinal.
De façon avantageuse, le moyen pharmaceutiquement acceptable de flottaison est un moyen sensiblement insoluble dans l'eau, en particulier insoluble en milieu gastrique. De manière plus spécifique, le moyen pharmaceutiquement acceptable de flottaison est un moyen sensiblement insoluble au moins dans la zone de pH comprise entre 3 et 7,5, avantageusement entre 2 et 7,5, de préférence entre 1 et 7,5. Lorsque la formulation est à usage humain, le moyen pharmaceutiquement acceptable de flottaison est un moyen apte à passer le transit humain sans ou sensiblement sans dégradation, par exemple des fibres aptes à passer le transit humain sans ou sensiblement sans dégradation.
Par exemple, le moyen de flottaison comporte un support, avantageusement sensiblement sphérique, apte à gonfler en présence d'eau.

Selon un détail d'une forme de réalisation, la formulation se présente sous forme de microsphères solides contenant de la bétaine, en particulier de la glycine bétaine, lesdites microsphères étant revêtues d'au moins une couche d'un polymère hydrophile apte à gonfler en milieu gastrique, mais insoluble en milieu gastrique. Les microsphères de bétaine (avant leur revêtement) ont par exemple une taille comprise entre 50 µm et 5 mm, avantageusement entre 100 µm et 2 mm, par exemple 250 µm à 1,5 mm, en particulier entre 500 µm et 1 mm. Le rapport en poids sec polymère hydrophile / microsphère de bétaine est avantageusement compris entre 0,5 et 10, avantageusement entre 1 et 8, par exemple entre 2 et 5.
De façon avantageuse, pour chaque microsphère, une couche sensiblement non gonflable et à libération contrôlée en milieu gastrique se situe entre la microsphère de bétaine et la couche de polymère hydrophile apte à gonfler en milieu gastrique. Ceci permet de contrôler la libération de la bétaine dans la couche de polymère gonflable une fois que cette couche a formée un gel entourant la sphère de bétaine.
Selon une forme de réalisation avantageuse, le polymère hydrophile apte à gonfler présente un taux de gonflement d'au moins 2, avantageusement d'au moins 4, en particulier d'au moins 5 à 20. Le taux de gonflement est défini comme étant le rapport entre le volume maximal d'un gramme de polymère dans de l'eau à pH 7 et le volume du polymère à l'état sec.
Selon un détail avantageux, chaque microsphère est associée à au moins une couche barrière imperméable à l'eau pour protéger au moins la couche de polymère hydrophile gonflable, ladite couche barrière étant soit soluble dans l'eau, en particulier à des pH inférieurs à 5, soit partiellement dégradable en milieu gastrique. Cette couche barrière permet ainsi d'éviter un gonflement du polymère hydrophile avant que les microsphères atteignent au moins l'estomac du patient. Lorsque la couche est soluble ou dégradable, elle est avantageusement telle qu'elle assure un effet barrière empêchant le gonflement du polymère hydrophile pendant au moins 30 secondes dans de l'eau à pH 5 et à 20°C. Par couche dégradable, on entend également une couche apte à fondre, telle qu'une couche à base de cire ou d'huile, en particulier d'huile végétale ou de dérivés de telles cires ou huiles.

De façon avantageuse, pour l'homme, ladite formulation comprend ou est associée à une quantité efficace d'au moins un moyen assurant une flottaison d'au moins une bétaine dans le tractus gastro-intestinal, de manière à assurer une flottaison d'au moins 50% en poids de la bétaine dans le tractus gastro-intestinal pendant au moins 3heures, de préférence pendant au moins 6 heures, en particulier pendant au moins 9 heures, plus spécifiquement pendant au moins 12 heures après l'administration. Selon une forme préférée, ladite formulation comprend ou est associée à une quantité efficace d'au moins un moyen assurant une flottaison d'au moins une bétaine dans le tractus gastro-intestinal, de manière à assurer une flottaison d'au moins 75% en poids de la bétaine dans le tractus gastro-intestinal pendant au moins 3 heures, de préférence pendant au moins 6 heures après l'administration. Selon une forme encore plus spécifique, ladite formulation comprend ou est associée à une quantité efficace d'au moins un moyen assurant une flottaison d'au moins une bétaine dans le tractus gastro-intestinal, de manière à assurer une flottaison d'au moins 85% en poids de la bétaine dans le tractus gastro-intestinal pendant au moins 3 heures après l'administration.

Selon un détail d'une formulation orale suivant l'invention, celle-ci comprend ou est associée à un polymère physiologiquement acceptable, de préférence un polymère hydrophile insoluble dans l'eau et insoluble en milieu gastrique, qui au contact des liquides et/ou des fluides gastriques est apte à former une forme, avantageusement gélatineuse et/ou gélifiée, dont la densité est inférieure à celle des fluides gastriques et/ou du contenu gastrique.

La formulation orale suivant l'invention peut se présenter sous la forme de capsules, de microcapsules, de comprimés, de tablettes, de sphéroïdes, de liquides, de gels ou de paillettes, éventuellement placés dans une enveloppe soluble au contact des sucs gastriques.

L'invention a encore pour objet l'utilisation d'au moins un moyen assurant une flottaison d'au moins une bétaine dans le tractus gastro-intestinal pour la préparation d'une formulation orale à libération contrôlée de bétaine, en particulier selon l'invention.

En particulier, l'invention a pour objet l'utilisation dudit moyen pour la préparation d'une formulation comprenant une bétaine et présentant au moins une caractéristique choisie parmi:
- augmenter le temps de résidence dans le tractus gastro-intestinal d'au moins une bétaine
- contrôler et/ou augmenter en fonction du temps le relargage d'au moins une bétaine vers le flux sanguin
- contrôler et/ou augmenter la quantité relarguée d'au moins une bétaine vers le flux sanguin
- contrôler et/ou augmenter la biodisponibilité d'au moins une bétaine, et
- combinaisons de telles caractéristiques.

Dans le champ de la présente invention diverses approches ont été poursuivies et revendiquées pour augmenter la rétention d'une bétaine dans le système gastro-intestinal soit : les systèmes équilibrés d'une façon hydrodynamique, systèmes non effervescents, systèmes effervescents, systèmes expansifs, systèmes bioadhésifs, systèmes à hautes densités, systèmes et appareils retardant la vidange gastrique et systèmes flottants.
Dans un aspect particulier les systèmes et formulations pH dépendant pourront être utilisées ainsi que les formes hydrodynamiquement balancées.

Les polymères et copolymères seuls ou en combinaison utilisés dans la présente invention peuvent être les suivants :
cellulose et ses dérivés, polyamide, polymère acrylique, polyvinylpyrrolidone, polyéthylène glycol, polystyrène, polyvinylalcohol, vinyle chloride-vinyl acétate copolymère, ethylène-vinyle acétate copolymère, vinyle chloride-propylène-vinyle acétate copolymère, polyvinylformal, polyvinvylacétate, polybutadiène, polyvinylbutyral, polyamides, polyimides, polymères acryliques, polyesters, polyvinylpyrrolidone, amidon, polyéthylène glycols, polystyrène, polyvinylalcohol, myristyl alcool et stéaryle alcool polymères, polyvinyle acétate, polybutadiène, polyvinyle formal, polyvinylbutyral, vinyle chloride-vinyl acétate copolymère, ethylène-vinyle acétate copolymère, vinyle chloride-propylène-vinyle acétate copolymère, les polysaccharides hydrocolloides, les polycarbonate, polyacrylate, polyméthacrylate, copolymère acrylate-méthacrylate, phthalate d'acétate de cellulose, la cellulose d'éthyle et la cellulose de méthyle hydroxypropyle, l'oxyde de polyéthylène, le glyceryl monostéarate, myristate de sodium, alkyl, la cellulose substituée, l'acide polyacrylique et leurs mixtures.

On pourra également utiliser des formulations à hautes densités afin d'avoir une densité plus grande que 1, ceci pour augmenter le temps de résidence gastrique. Ceci peut être atteint en revêtant le médicament avec un matériel inerte lourd tel que sulfate de baryum, l'oxyde de zinc, le dioxyde de titanium etc.
Dans un aspect de l'invention des polymères pourront être utilisés afin d'augmenter le temps de résidence dans le tractus gastro-intestinal d'au moins une bétaine. Les bétaines pouvant être liées, synthétisées et/ou combinées à ces polymères afin d'augmenter leurs temps de résidence dans le tractus gastro-intestinal, et formulées dans des formes flottantes ou non.

Dans un aspect particulier de l'invention des polymères muco-adhésifs pourront être utilisés afin d'augmenter le temps de résidence dans le tractus gastro-intestinal d'au moins une bétaine. Les bétaines pouvant être liées, synthétisées et/ou combinées à ces polymères muco-adhésifs afin d'augmenter leurs temps de résidence dans le tractus gastro-intestinal, et formulées dans des formes flottantes ou non.

Les lubrifiants qui peuvent être utilisés de manière non limitative sont les stéarates d'aluminium, le calcium, le magnésium, l'étain, les silicates de magnésium, les stéarates de magnésium, les thixcins, les silicones et substances similaires et leurs mixtures.

Les substances possibles facilitant le flux qui peuvent être utilisées de manière non limitative sont le talc, les colloïdes de silice, l'amidon, les microcristaux de cellulose et substances similaires et leurs mixtures.

Les binders qui peuvent être utilisés de manière non limitative amidons, alginates, carboxymethylcellulose ou polyvinylpyrrolidone et substances similaires et leurs mixtures.

Les agents de bulking ou de consistance qui peuvent être ajoutés sont, par exemple, les agents de bulking inorganiques tel que les oxydes de magnésium et/ou d'aluminium et/ou de silicium, l'aluminium, le silicium, ou le carbonate de titane ou carbonate de calcium, bicarbonate de sodium, les phosphates, chlorures de sodium et substances similaires et leurs mélanges.

Dans un aspect particulier de l'invention, le carbonate de calcium, bicarbonate de sodium, les phosphates, chlorures de sodium, leurs esters et substances similaires et leurs mélanges pourront être utilisés afin d'augmenter la biodisponibilité dans le tractus gastro-intestinal d'au moins une bétaine.

Dans le cadre de la présente invention la libération d'au moins une bétaine peut être accélérée par l'ajout de polymères qui sont librement solubles dans l'eau, et peut être également ralentie en ajoutant des substances qui sont très lipophiles ou des substances qui enflent dans l'eau ce qui mène à la formation de gel dans les pores de la matrice inerte et ainsi empêche la diffusion de cette bétaine en dehors de ladite matrice. Les exemples de substances formant de tels gels peuvent être dans le cadre de la présente invention les alginates, pectines, galactomannanes, carrageenans, dextrans, curdlan, pullulan, gellan, chitines, la gélatine, xanthates, hémicelluloses, les dérivées de cellulose tel que methylcellulose, hydroxypropylmethylcellulose, hydroxypropylcellulose, hydroxyéthylcellulose, carboxymethylcellulose, dérivés d'amidon tel que l'amidon de carboxymethyl, l'amidon dégradé, maltodextrins, l'acide de polyacrylique, l'acide de polymethacrylique, l'acide méthacrylique d'acrylique copolymères acide, les alcools de polyvinyle, les glycols de polyéthylène de hauts poids moléculaires, les copolymères de polyoxyethylene/ polyoxypropylene, hauts polyvinylpyrrolidones de poids moléculaires et leurs dérivés. Les substances lipophiles incluent, par exemple, les alcools gras tel que l'alcool stéaryle, les acides gras tel que l'acide stéarique, glycérides, esters acide gras et esters d'alcool gras, les polymères lipophiles tel que l'ethylcellulose, l'acétate de cellulose, l'ester méthacrylique ester copolymères acrylique, méthacrylique ester copolymères acide acrylique, phthalate d'acétate de cellulose, succinate d'acétate de cellulose, phthalate d'acétate de hydroxypropylmethylcellulose, succinate d'acétate d'hydroxypropylmethylcellulose. Les polymères solubles dans l'eau incluent, par exemple, glycols de polyéthylène, polyvinylpyrrolidone ou copolymères d'acétate de vinylpyrrolidone vinyle.

Les colorants qui peuvent être ajoutés sont des oxydes de fers, le dioxyde de titanium, les teintures de triphénylméthane, les teintures d'azo, les teintures de quinoléine, les teintures d'indigo, les caroténoïdes, les agents opacifiants tel que le dioxyde de titanium ou tel que talc pour augmenter la transparence ou pour préserver les colorants.

Les substances qui augmentent la perméabilité de la membrane intestinale, connues de l'homme de l'art, pourront également être utilisées dans le cadre de la présente invention, afin d'optimiser la quantité de passage d'au moins une bétaine vers le sang. Les activateurs d'absorption pouvant être des gommes, des résines, des sels, ainsi que toute substance connue de l'homme du métier à cet effet.

Selon l'invention, les moyens tels que décrits dans la présente demande pourront être combinés afin d'optimiser l'invention et l'efficacité des compositions et/ou formes revendiquées.

Les formes telles que revendiquées dans la présente invention pourront augmenter le temps de résidence dans le tractus gastro-intestinal, d'au moins une bétaine, pendant 3 heures, 6 heures, 12 heures, 24 heures, 48 heures, 72 heures, 96 heures, une semaine, un mois.

Les gommes telles que celles issues du gellan, gomme carragheen, gomme karaya, gomme tragacanth, gomme ghatti, glucomannan, gomme guar, gomme acacia, gomme haricot locuste, gomme xanthan, veegum, gomme gellan et dérivées tragacanth cellulose like, hydroxy propyl methyl cellulose , hydroxy propyl cellulose , carboxymethyl cellulose et ses dérivées, pectines, lignine, chitines et ses dérivées, acides acryliques, agar, gélatine, alcool polyvinyle , carbomères tels que les carbopols, ou leurs combinaisons pourront être utilisées comme bioadhésifs et/ou substances améliorant le passage d'au moins une bétaine au départ du tractus gastro-intestinal vers la circulation sanguine.

Les substances générant un gaz peuvent produire le dioxyde de carbone ou le dioxyde de soufre au contact avec le liquide gastrique. Les exemples de générateurs de gaz qui peuvent être utilisés dans l'invention incluent les générateurs de dioxyde de carbone, tel que carbonate de calcium ou le sodium glycine carbonate, le bicarbonate tel que carbonate d'hydrogène de sodium ou carbonate d'hydrogène de potassium, les générateurs de dioxyde de Soufre tel que sulfite de sodium, bisulfite de sodium, metabisulfite de sodium, et les substances similaires.
Le générateur de gaz réagit par simple contact avec le liquide gastrique pour engendrer le dioxyde de carbone ou le dioxyde de soufre qui est emprisonné dans la matrice ou le gel de la forme flottante, qui sous cet effet augmente de volume flotte au dessus des fluides gastriques et libère de manière contrôlée au mois une bétaine.
Selon l'invention la composition flottante peut contenir des diluants solubles ou insolubles et de manière préférentielle dans l'eau. Les exemples de diluants solubles dans l'eau qui peuvent être utilisés dans l'invention incluent mais de manière non limitative le lactose, sucrose, mannitol et substances similaires. Les exemples de diluants insolubles qui peuvent être utilisés dans l'invention actuelle incluent mais de manière non limitative le phosphate de calcium dibasique, l'amidon et les microcristaux de cellulose.

Les binders ou liants pharmaceutiquement acceptables tels que Polyvinylpyrrolidone 30/90, Hydroxypropyl methylcellulose, et hydroxypropylcellulose peuvent être utilisés dans la formulation actuelle. En plus des ingrédients ci-dessus mentionnés, on pourra utiliser le dioxyde de silicium colloïdal (Aerosil 200) comme lubrifiant, inclus dans la matrice. Préférablement le stéarate de magnésium, l'acide stéarique, talc et le dioxyde de silicium colloïdal ,seuls ou en combinaisons peuvent être utilisés. On peut également inclure des antioxidants.
Les tablettes peuvent être revêtues d'un film qui se dissous rapidement afin de former le polymère soluble dans l'eau comme l'hydroxypropyl methylcellulose, l'acrylate, la cellulose d'éthyle et/ou des excipients solubles dans l'eau.
Dans un autre aspect de l'invention les formes pourront être multi- compartimentées avec des profils et/ou des temps de désintégration différents. Ces compartiments avec des profils et/ou des temps de désintégration différents permettront, en cas de saturation du site d'absorption au niveau du tractus gastro-intestinal d'au moins une bétaine, de contrôler la quantité et/ou le moment où cette bétaine sera libérée afin qu'une absorption optimale se fasse sur ledit site. Dans un aspect de l'invention ces compartiments peuvent remplacés par des couches successives de bétaines et de principes assurant la flottabilité et/ou l'adhérence dans le tractus gastro-intestinal.

Les termes " bétaine" et/ou " bétaines" employés dans la présente invention se réfèrent aux composés choisis dans le groupe consistant en des bétaines lipidiques, des lipides de bétaine, et/ou des bétaines de formule (CH₃)₃N⁺- (CH₂)ₙ - COO⁻ avec n un nombre entier de 1 à 5, (préférablement la glycine bétaine n = 1), leurs sels pharmaceutiquement acceptables, leurs esters, leurs précurseurs et leurs mélanges. Les termes " bétaines lipidiques " et " lipides de bétaine " se réfèrent aux lipides de bétaine et/ou bétaines lipidiques qui sont des éléments structuraux des membranes que l'on trouve communément dans les fougères, les mousses, les mycètes, les champignons, les amibes, les eucaryotes, les plantes sauvages et les algues. Les lipides de bétaine sont des glycérolipides non phosphoriques éthers liés, qui ressemblent par leur structure générale à la plus communément connue phosphatidylcholine. Les plus communs des glycérolipides contiennent une portion de diacyl glycérol à laquelle un groupe polaire est attaché. Ce groupe polaire peut être une portion carbohydrate comme dans les galactolipides très abondants dans les plantes ou un phosphorylester comme dans les glycéro phospholipides, la classe lipidique la plus commune chez les animaux. Les lipides de bétaine représentent une troisième classe de glycérolipides dans lequel un alcool amine quaternaire est lié dans une liaison éther à la portion diacylglycerol. Ils peuvent être obtenus par extraction, biosynthèse ou par synthèse. Les lipides de bétaines diacylglyceryl- O-4' - (N, N, N- trimethyl) homoserine et une proche isoforme le diacylglyceryl -O-2'-(hydroxymethyl) (N, N, N- trimethyl)-β-alanine sont les plus communs.

Dans un aspect particulier de l'invention ces formes lipidiques pourront être préférées, du à leur meilleure biodisponibilité.

Dans un aspect particulier de l'invention les bétaines pourront être combinées ou synthétisées avec des lipides, des huiles ou des graisses et leurs mélanges.

Dans un aspect particulier de l'invention la forme flottante pourra être sous forme liquide, ladite forme liquide se gonflant au contact des sucs et/ou acides de l'environnement gastro-intestinal. Ce gonflement modifiant la densité de ladite forme liquide afin de lui assurer la flottabilité.

Dans un aspect de l'invention les formes pharmaceutiques, pouvant être hydrodynamiquement balancées ou non, qui peuvent être réalisées appartiennent à la classe des matrices hydrophiles monolithiques. Ces formes matricielles sont non-désintégrantes et non-digérables. La formulation homogène, contenue au départ dans une capsule de gélatine dure, est préparée à partir d'un ou plusieurs polymères hydrophiles.

Les formes décrites peuvent être combinées afin de réaliser l'invention.

### Exemples

### Exemple 1

### Comprimé flottant de bétaine

1,6 kg de bétaine, 1,6 kg de mélange formulé d'acétate de polyvinyle et polyvinylpyrrolidone dans la proportion 8:2 (Kollidon SR) et 0,02 kg de stéarate de magnésium a été passé par un tamis de 0,8 mm, mélangé dans un mélangeur Turbula pendant 10 min. puis compressé dans une presse excentrique Korsch EKO afin de réaliser des comprimés biplans d'un poids de 650 mg. La force de compressive était 3,04 kN.

Les comprimés ont flotté immédiatement après addition à un fluide gastrique simulé. La flottaison a persisté pendant 38 h.

| Profil de libération d'un comprimé flottant | |
|---|---|
| Temps (h) | Quantité libérée (%) |
| 0 | 0 |
| 1 | 15,6 |
| 2 | 23,1 |
| 4 | 37,7 |
| 8 | 52,3 |
| 12 | 65,0 |
| 18 | 83,6 |
| 24 | 98,2 |

### Exemple 2

### Comprimé hydroxypropylmethylcellulose flottant de bétaine

1,6 kg de bétaine, 1,6 kg de hydroxypropylmethylcellulose (Methocel K 100) et 0,02 kg de stéarate de magnésium a été passé par un tamis de 0,8 mm, mélangé dans un mélangeur Turbula pendant 10 min. puis compressé dans une presse excentrique Korsch EKO afin de réaliser des comprimés biplans d'un poids de 650 mg. La force de compressive était 2,05 kN.

Les comprimés ont flotté immédiatement après addition à un fluide gastrique simulé. La flottaison a persisté pendant 24 h.

| Profil de libération d'un comprimé flottant | |
|---|---|
| Temps (h) | Quantité libérée (%) |
| 0 | 0 |
| 1 | 12,7 |
| 2 | 21,4 |
| 4 | 36,2 |
| 8 | 58,3 |
| 12 | 66,9 |
| 18 | 79,5 |
| 24 | 92,6 |

### Exemple 3

### Comprimé Ester copolymère Acrylique flottant de bétaine

1,6 kg de bétaine, 1,6 kg Eudragit RS et 0,02 kg de stéarate de magnésium a été passé par un tamis de 0,8 mm, mélangé dans un mélangeur Turbula pendant 10 min. puis compressé dans une presse excentrique Korsch EKO afin de réaliser des comprimés biplans d'un poids de 650 mg. La force de compressive était 5,05 kN.
Les comprimés ont flotté immédiatement après addition à un fluide gastrique simulé. La flottaison a persisté pendant 37 h.

| Profil de libération d'un comprimé flottant | |
|---|---|
| Temps (h) | Quantité libérée (%) |
| 0 | 0 |
| 1 | 13,9 |
| 2 | 22,6 |
| 4 | 40,3 |
| 8 | 61,8 |
| 12 | 75,9 |
| 18 | 84,9 |
| 24 | 98,0 |

### Exemple 4

### Préparation d'un granulé à libération prolongée.

On effectue un mélange comprenant 38% d'un comprimable Eudragit RPL RTM , 60% de bétaine anhydre, 1% de stearate de talc et 1% magnésium. Après traitement dans un mélangeur Turbula T2C, le mélange a été compacté au moyen d'un appareil EKO de Korsch à 40,000 N/cm², puis a été transformé en granulés au moyen d'un granuleuse Erweka TG II S. Les granulés ont été ensuite triés au moyen d'une vibreuse ne gardant que les fractions ayant un diamètre entre 100 et 150. µm. Les granulés ainsi obtenus pouvant enter dans diverses préparations notamment des gélules, des comprimés ou des gels.

### Exemple 5

De la bétaine en poudre (granulométrie de moins de 250 µm, en particulier de moins de 100 µm) a été mélangé à une huile ou cire ou un alcool solide à température ambiante, par exemple de l'huile de coco ou de l'alcool stéarylique. Pour réaliser ce mélange, on a fondu l'huile, l'alcool ou cire pour obtenir une forme liquide. On a ajouté la bétaine en poudre à cette huile, cire ou alcool fondu. On a fait varier le rapport en poids betaine / huile ou cire ou alcool entre 0,05 et 2 (les rapports inférieurs à 1 étant préférés, tels que 0,2, 0,3, 0,4 et 0,5).

On transforme ce mélange liquide en microsphère, par exemple par pulvérisation ou par extrusion - sphéronisation, etc. Les microsphères ainsi préparées ont une taille de 500 µm, 750 µm, et 1 mm.

Ces microsphères ont été revêtues en une ou plusieurs étapes, d'une couche d'un polymère hydrophile gonflable à l'eau, mais insoluble en milieu gastrique. On a fait varier le rapport en poids sec polymère hydrophile / microsphère de bétaine entre 1 et 10.

Les billes ainsi obtenues ont ensuite été munies d'une fine couche d'un polymère insoluble à l'eau, mais soluble ou dégradable en milieu gastrique, par exemple un revêtement polyacrylate Eudragit.

## Revendications

1. Formulation orale à libération contrôlée destinée à libérer au moins une bétaine après administration orale, **caractérisée en ce que** ladite formulation comprend ou est associée à au moins un moyen pharmaceutiquement acceptable assurant une flottaison d'au moins une bétaine dans le tractus gastro-intestinal.

2. Formulation suivant la revendication 1, **caractérisée en ce que** le moyen pharmaceutiquement acceptable de flottaison est un moyen sensiblement insoluble dans l'eau.

3. Formulation suivant la revendication 1, **caractérisée en ce que** le moyen pharmaceutiquement acceptable de flottaison est un moyen sensiblement insoluble en milieu gastrique.

4. Formulation suivant la revendication 1, **caractérisée en ce que** le moyen pharmaceutiquement acceptable de flottaison est un moyen sensiblement insoluble au moins dans la zone de pH comprise entre 3 et 7,5, avantageusement entre 2 et 7,5, de préférence entre 1 et 7,5.

5. Formulation suivant la revendication 4 à usage humain, **caractérisée en ce que** le moyen pharmaceutiquement acceptable de flottaison est un moyen apte à passer le transit humain sans ou sensiblement sans dégradation.

6. Formulation suivant la revendication 5, **caractérisée en ce que** le moyen de flottaison comporte des fibres aptes à passer le transit humain sans ou sensiblement sans dégradation.

7. Formulation suivant la revendication 5, **caractérisée en ce que** le moyen de flottaison comporte un support, avantageusement sensiblement sphérique, apte à gonfler en présence d'eau.

8. Formulation suivant l'une quelconque des revendications précédentes, **caractérisée en ce qu'**elle se présente sous la forme de microsphères solides contenant de la bétaine, en particulier de la glycine bétaine, lesdites microsphères étant revêtues d'au moins une couche d'un polymère hydrophile apte à gonfler en milieu gastrique, mais insoluble en milieu gastrique.

9. Formulation suivant la revendication 8, **caractérisée en ce que** pour chaque microsphère, une couche sensiblement non gonflable et à libération contrôlée en milieu gastrique se situe entre la microsphère de bétaine et la couche de polymère hydrophile apte à gonfler en milieu gastrique.

10. Formulation suivant la revendication 8 ou 9, **caractérisée en ce que** le polymère hydrophile apte à gonfler présente un taux de gonflement d'au moins 2, avantageusement d'au moins 4, en particulier d'au moins 5 à 20.

11. Formulation suivant l'une quelconque des revendications 8 à 10, **caractérisée en ce que** chaque microsphère est associée à au moins une couche barrière imperméable à l'eau pour protéger au moins la couche de polymère hydrophile gonflable, ladite couche barrière étant soit soluble dans l'eau, soit partiellement dégradable en milieu gastrique.

12. Formulation orale suivant la revendication 1 pour l'homme, **caractérisée en ce que** ladite formulation comprend ou est associée à une quantité efficace d'au moins un moyen assurant une flottaison d'au moins une bétaine dans le tractus gastro-intestinal, de manière à assurer une flottaison d'au moins 50% en poids de la bétaine dans le tractus gastro-intestinal pendant au moins 3 heures, de préférence pendant au moins 6 heures, en particulier pendant au moins 9 heures, plus spécifiquement pendant au moins 12 heures après l'administration.

13. Formulation orale suivant la revendication 12, **caractérisée en ce que** ladite formulation comprend ou est associée à une quantité efficace d'au moins un moyen assurant une flottaison d'au moins une bétaine dans le tractus gastro-intestinal, de manière à assurer une flottaison d'au moins 85% en poids de la bétaine dans le tractus gastro-intestinal pendant au moins 3 heures après l'administration.

14. Formulation orale suivant la revendication 1, **caractérisée en ce que** ladite formulation comprend ou est associée à une quantité efficace d'au moins un moyen assurant une flottaison d'au moins une bétaine dans le tractus gastro-intestinal, de manière à assurer une flottaison d'au moins 75% en poids de la bétaine dans le tractus gastro-intestinal pendant au moins 3 heures, de préférence pendant au moins 6 heures après l'administration.

15. Formulation orale suivant l'une quelconque des revendications précédentes, **caractérisée en ce qu'**elle comprend ou est associée à un polymère physiologiquement acceptable, de préférence un polymère hydrophile insoluble dans l'eau et insoluble en milieu gastrique, qui au contact des liquides et/ou des fluides gastriques est apte à former une forme, avantageusement gélatineuse et/ou gélifiée, dont la densité est inférieure à celle des fluides gastriques et/ou du contenu gastrique.

16. Formulation orale suivant l'une quelconque des revendications précédentes, **caractérisée en ce qu'**elle se présente sous la forme de capsules, de microcapsules, de comprimés, de tablettes, de sphéroïdes, de liquides, de gels ou de paillettes, éventuellement placés dans une enveloppe soluble au contact des sucs gastriques.

17. Utilisation d'au moins un moyen assurant une flottaison d'au moins une bétaine dans le tractus gastro-intestinal pour la préparation d'une formulation orale à libération contrôlée de bétaine, en particulier selon l'une quelconque des revendications 1 à 16.

18. Utilisation suivant la revendication 17 pour la préparation d'une formulation comprenant une bétaine et présentant au moins une caractéristique choisie parmi:
- augmenter le temps de résidence dans le tractus gastro-intestinal d'au moins une bétaine
- contrôler et/ou augmenter en fonction du temps le relargage d'au moins une bétaine vers le flux sanguin
- contrôler et/ou augmenter la quantité relarguée d'au moins une bétaine vers le flux sanguin
- contrôler et/ou augmenter la biodisponibilité d'au moins une bétaine, et
- combinaisons de telles caractéristiques.

## Claims

1. Oral control release formulation for releasing at least one betaine after oral administration, **characterized in that** said formulation comprises or is associated to at least one pharmaceutically acceptable means ensuring a floating of at least one betaine in the gastro-intestinal tractus.

2. Formulation according to claim 1, **characterized in that** the pharmaceutically acceptable floating means is a substantially water insoluble means.

3. Formulation according to claim 1, **characterized in that** the pharmaceutically acceptable floating means is a substantially gastric insoluble means.

4. Formulation according to claim 1, **characterized in that** the pharmaceutically acceptable floating means is a means substantially insoluble at least in the pH range comprised between 3 and 7.5, advantageously between 2 and 7.5, preferably between 1 and 7.5.

5. Formulation for human use according to claim 4, **characterized in that** the pharmaceutically acceptable floating means is a means suitable to pass the human transit without or substantially without degradation.

6. Formulation according to claim 5, **characterized in that** the floating means comprises fibers adapted for passing the human transit without or substantially without degradation.

7. Formulation according to claim 5, **characterized in that** the floating means comprises a support, advantageously substantially spherical, able to swell in the presence of water.

8. Formulation according to any one of the preceding claims, **characterized in that** it has the form of solid micro-spheres containing betaine, in particular glycine betaine, said micro-spheres being coated with at least one layer of a hydrophilic polymer able to swell in gastric medium, but insoluble in gastric medium.

9. Formulation according to claim 8, **characterized in that** for each micro-sphere, a controlled release layer that is substantially non swelling in gastric medium is located between the betaine micro-sphere and the gastric medium.

10. Formulation according to claims 8 or 9, **characterized in that** the hydrophilic polymer able to swell has a swelling rate of at least 2, advantageously at least 4, preferably comprised between 5 and 20.

11. Formulation according to any one of claims 8 to 10, **characterized in that** each micro-sphere is associated to at least one water impermeable barrier layer for protecting at least the hydrophilic swelling polymer layer, said barrier layer being soluble in water or partially degradable in gastric medium.

12. Oral formulation for human according to claim 1, **characterized in that** said formulation comprises or is associated to an efficient amount of at least one means ensuring a floating of at least one betaine in the gastro intestinal tractus, so as to ensure the floating of at least 50% by weight of the betaine in the gastro-intestinal tractus for at least 3 hours, preferably for at least 6 hours, in particular for at least 9 hours, more specifically for at least 12 hours after the administration.

13. Oral formulation according to claim 12, **characterized in that** said formulation comprises or is associated to an efficient amount of at least one means ensuring the floating of at least one betaine in the gastro-intestinal tractus, so as to ensure the floating of at least 85% by weight of the betaine in the gastro-intestinal tractus for at least 3 hours after the administration.

14. Oral formulation according to claim 1, **characterized in that** said formulation comprises or is associated to an efficient amount of at least one means ensuring the floating of at least one betaine in the gastro-intestinal tractus, so as to ensure a floating of at least 75% by weight of the betaine in the gastro-intestinal tractus for at least 3 hours, preferably for at least 6 hours after the administration.

15. Oral formulation according to any one of the preceding claims, **characterized in that** it comprises or is associated to a physiologically acceptable polymer, preferably water insoluble hydrophilic polymer insoluble in gastric medium, which in contact with gastric liquids and/or gastric fluids is able to form a form, advantageously gelatinous and/or jellified, the density of which is lower than that of the gastric fluids and/or of the gastric content.

16. Oral formulation according to any one of the preceding claims, **characterized in that** it has the form of capsules, microcapsules, tablets, spheroids, liquids, gels, flakes, possibly placed in an envelope soluble in contact with the gastric juices.

17. Use of at least one means ensuring a floating of at least one betaine in the gastro-intestinal tractus for the preparation of an oral controlled release formulation of betaine, in particular according to any one of the claims 1 to 16.

18. Use according to claim 17 for the preparation of a formulation comprising a betaine and having at least one characteristic selected from:
- increasing the residence time in the gastro-intestinal tractus of at least one betaine
- control and/or increase in function of time the passage of at least one betaine towards the blood flow
- control and/or increase the released amount of at least one betaine towards the blood flow
- control and/or increase the bioavailability of at least one betaine, and
- combinations of said characteristics.

## Patentansprüche

1. Orale Formulierung mit kontrollierter Freisetzung, die dazu bestimmt ist, wenigstens ein Betain nach oraler Verabreichung freizusetzen, **dadurch gekennzeichnet, dass** die Formulierung wenigstens ein pharmazeutisch annehmbares Mittel umfasst oder mit wenigstens einem pharmazeutisch annehmbaren Mittel assoziiert ist, welches eine Flotation wenigstens eines Betains im gastrointestinalen Trakt sicherstellt.

2. Formulierung nach Anspruch 1, **dadurch gekennzeichnet, dass** das pharmazeutisch annehmbare Flotationsmittel ein Mittel ist, das in Wasser deutlich unlöslich ist.

3. Formulierung nach Anspruch 1, **dadurch gekennzeichnet, dass** das pharmazeutisch annehmbare Flotationsmittel ein Mittel ist, das im Magenmilieu deutlich unlöslich ist.

4. Formulierung nach Anspruch 1, **dadurch gekennzeichnet, dass** das pharmazeutisch annehmbare Flotationsmittel ein Mittel ist, das wenigstens in der pH-Zone zwischen 3 und 7,5, vorteilhafterweise zwischen 2 und 7,5, vorzugsweise zwischen 1 und 7,5, deutlich unlöslich ist.

5. Formulierung nach Anspruch 4 zur menschlichen Verwendung, **dadurch gekennzeichnet, dass** das pharmazeutisch annehmbare Flotationsmittel ein Mittel ist, das geeignet ist, die humane Passage ohne Abbau oder ohne merklichen Abbau zu passieren.

6. Formulierung nach Anspruch 5, **dadurch gekennzeichnet, dass** das Flotationsmittel Fasern enthält, die fähig sind, die humane Passage ohne Abbau oder ohne merklichen Abbau zu passieren.

7. Formulierung nach Anspruch 5, **dadurch gekennzeichnet, dass** das Flotationsmittel einen Träger, der vorteilhafterweise etwa sphärisch ist, der geeignet ist, in Gegenwart von Wasser zu quellen, umfasst.

8. Formulierung nach einem der vorangehenden Ansprüche, **dadurch gekennzeichnet, dass** sie sich in Form von festen Mikrokügelchen präsentiert, die Betain, insbesondere Glycinbetain, enthalten, wobei die Kügelchen von wenigstens einer Schicht eines hydrophilen Polymers überzogen sind, das fähig ist, im Magenmilieu aufzuquellen, im Magenmilieu aber unlöslich ist.

9. Formulierung nach Anspruch 8, **dadurch gekennzeichnet, dass** sich für jedes Mikrokügelchen eine nicht merklich quellbare Schicht mit kontrollierter Freisetzung im Magenmedium zwischen dem Betainmikrokügelchen und der Schicht aus hydrophilem Polymer, die fähig ist, im Magenmilieu zu quellen, befindet.

10. Formulierung nach Anspruch 8 oder 9, **dadurch gekennzeichnet, dass** das hydrophile Polymer, das fähig ist, zu quellen, einen Quellgrad von wenigstens 2, vorzugsweise wenigstens 4, insbesondere wenigstens 5 bis 20 aufweist.

11. Formulierung nach einem der Ansprüche 8 bis 10, **dadurch gekennzeichnet, dass** jedes Mikrokügelchen mit wenigstens einer für Wasser undurchlässigen Sperrschicht assoziiert ist, um wenigstens die Schicht aus quellbarem hydrophilem Polymer zu schützen, wobei die Sperrschicht entweder in Wasser löslich ist oder im Magenmedium partiell abbaubar ist.

12. Orale Formulierung nach Anspruch 1 für den Menschen, **dadurch gekennzeichnet, dass** die Formulierung eine wirksame Menge wenigstens eines Mittels enthält oder damit assoziiert ist, welche eine Flotation wenigstens eines Betains im Gastrointestinaltrakt sicherstellt, derart, dass eine Flotation von wenigstens 50 Gew.-% des Betains im Gastrointestinaltrakt während wenigstens 3 Stunden, vorzugsweise während wenigstens 6 Stunden, insbesondere während wenigstens 9 Stunden, spezifischer während wenigstens 12 Stunden nach Verabreichung sichergestellt wird.

13. Orale Formulierung nach Anspruch 12, **dadurch gekennzeichnet, dass** die Formulierung eine wirksame Menge wenigstens eines Mittels umfasst oder damit assoziiert ist, die eine Flotation wenigstens eines Betains im Gastrointestinaltrakt sicherstellt, derart, dass eine Flotation von wenigstens 85 Gew.-% des Betains im Gastrointestinaltrakt während wenigsten 3 Stunden nach der Verabreichung sichergestellt wird.

14. Orale Formulierung nach Anspruch 1, **dadurch gekennzeichnet, dass** die Formulierung eine wirksame Menge wenigstens eines Mittels enthält oder damit assoziiert ist, die eine Flotation wenigstens eines Betains im Gastrointestinaltrakt sicherstellt, derart, dass eine Flotation von wenigstens 75 Gew.-% des Betains im Gastrointestinaltrakt während wenigstens 3 Stunden, vorzugsweise während wenigstens 6 Stunden nach der Verabreichung sichergestellt wird.

15. Orale Formulierung nach einem der vorangehenden Ansprüche, **dadurch gekennzeichnet, dass** sie ein physiologisch annehmbares Polymer enthält oder damit assoziiert ist, vorzugsweise ein hydrophiles Polymer, das in Wasser unlöslich ist und im Magenmilieu unlöslich ist, das im Kontakt mit Lipiden und/oder Magensäften fähig ist, eine Form, vorzugsweise eine gelatinöse und/oder gelierte Form, zu bilden, deren Dichte unter derjenigen der Magensäfte und/oder des Mageninhalts liegt.

16. Orale Formulierung nach einem der vorangehenden Ansprüche, **dadurch gekennzeichnet, dass** sie sich in Form von Kapseln, Mikrokapseln, Tabletten, Pastillen, Sphäroiden, Flüssigkeiten, Gelen oder Pailletten, die gegebenenfalls in eine Umhüllung platziert wurden, welche im Kontakt mit Magensaft löslich ist, präsentieren.

17. Verwendung wenigstens eines Mittels, das eine Flotation wenigstens eines Betains im Gastrointestinaltrakt sicherstellt, für die Herstellung einer oralen Betain-Formulierung mit kontrollierter Freisetzung, insbesondere nach einem der Ansprüche 1 bis 16.

18. Verwendung nach Anspruch 17 zur Herstellung einer Formulierung, die ein Betain enthält und wenigstens ein Merkmal aufweist, das ausgewählt ist unter:
- Erhöhen der Verweilzeit wenigstens eines Betains im Gastrointestinaltrakt,
- Kontrollieren und/oder Erhöhen der Abgabe wenigstens eines Betains in den Blutfluss als Funktion der Zeit,
- Kontrollieren und/oder Erhöhen der abgegebenen Menge wenigstens eines Betains in den Blutfluss,
- Kontrollieren und/oder Erhöhen der Bioverfügbarkeit wenigstens eines Betains und
- Kombinationen solcher Merkmale.
